(19) Europäisches Patentamt
European Patent Office
Office européen des brevets

(11) **EP 4 260 801 A2**

(12) **EUROPEAN PATENT APPLICATION**

(43) Date of publication:
**18.10.2023 Bulletin 2023/42**

(51) International Patent Classification (IPC):
**A61B 5/024** (2006.01)

(21) Application number: **23190658.7**

(52) Cooperative Patent Classification (CPC):
**A61B 5/0295; A61B 5/0077; A61B 5/02416; A61B 5/7203; A61B 5/7246;** A61B 2505/01

(22) Date of filing: **12.05.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.05.2016 US 201662335289 P**

(62) Document number(s) of the earlier application(s) in accordance with Art. 76 EPC:
**17725113.9 / 3 454 731**

(71) Applicant: **William Marsh Rice University Houston, TX 77005-1827 (US)**

(72) Inventors:
• **KUMAR, Mayank Houston, 77005-1827 (US)**
• **VEERARAGHAVAN, Ashok Houston, 77005-1827 (US)**
• **SABHARWAL, Ashutosh Houston, 77005-1827 (US)**

(74) Representative: **Osha Liang 2, rue de la Paix 75002 Paris (FR)**

Remarks:
This application was filed on 09.08.2023 as a divisional application to the application mentioned under INID code 62.

(54) **HIGH RESOLUTION BLOOD PERFUSION IMAGING USING A CAMERA AND A PULSE OXIMETER**

(57) In one aspect, embodiments disclosed herein relate to multi-sensor imaging systems for generating a pulsatile blood perfusion map, comprising a high accuracy blood flow sensor (702; 802) configured to generate a reference blood volume waveform at a reference site of a patient's body (706; 806), one or more low accuracy blood flow sensors (704; 804) configured to simultaneously generate a second blood volume waveform at any region of interest of the patient's body (708; 808), and a controller. The high accuracy blood flow sensor (702; 802) is a pulse oximeter and the one or more low accuracy blood flow sensors (704; 804) are one or more camera sensors each equipped with an optical filter.

FIG. 7

**Description**

BACKGROUND

Blood perfusion is the flow of blood to the end organs and tissues through the blood vessels in the body. Blood flow (or perfusion) is vital in ensuring oxygen delivery to the cells and in maintaining metabolic homeostasis. Blood perfusion generally varies from one tissue site to another, and can also changes over time due to varying metabolic demands, and so a spatial map of blood perfusion over time, i.e. a three-dimensional quantity, is measured. For example, dynamic changes in local perfusion happen due to changes in body's physiology during tissue repair, circulatory shock, and wound healing. Measuring reference perfusion, i.e., perfusion of the blood just underneath the skin surface is important in both medical and surgical fields, including assessment of reference perfusion in critical care, tissue viability in plastic, reconstructive, and burn surgery, as well as for wound assessment.

SUMMARY

This summary is provided to introduce a selection of concepts that are further described below in the detailed description. This summary is not intended to identify key or essential features of the claimed subject matter, nor is it intended to be used as an aid in limiting the scope of the claimed subject matter.

In one aspect, embodiments disclosed herein relate to a multi-sensor imaging system for generating a pulsatile blood perfusion map as defined in claim 1.

In another aspect, embodiments of the present disclosure relate to a method for generating pulsatile blood perfusion maps as defined in claim 8.

Further embodiments of the system and method are defined in the dependent claims.

In a further aspect, embodiments disclosed herein relate to a multi-sensor imaging system for measuring a pulsatile blood perfusion map, including: one or more high accuracy blood flow sensors that generate a reference blood volume waveform; one or more low accuracy blood flow sensors that generate a second blood volume waveform; and a controller connected to the high accuracy blood flow sensor and the one or more low accuracy blood flow sensors by at least one operable connection, wherein the controller is configured to generate the pulsatile blood perfusion map by analyzing the reference blood volume waveform and the second blood volume waveform.

Embodiments are directed to the system of paragraph [0006], wherein the one or more high accuracy blood flow sensors are placed at a reference site of a patient.

Embodiments are directed to the system of paragraph [0007], wherein the one or more high accuracy blood flow sensors are one or more selected from a group consisting of pulse oximeter, electrocardioagraph, arterial catheters, and camera-based photo-plethysmography device.

Embodiments are directed to the system of paragraph [0006], wherein the one or more high accuracy blood flow sensors and the one or more low accuracy blood flow sensors are referring to same or different fields of view of a same physical camera device.

Embodiments are directed to the system of paragraph [0006], wherein the one or more low accuracy blood flow sensors measure blood flow over reference and/or imaging sites of a body of the patient.

Embodiments are directed to the system of paragraph [0010], wherein the imaging sites are internal or external sites of the body of the patient.

Embodiments are directed to the system of paragraph [0010], wherein the one or more low accuracy blood flow sensors are physical devices that include at least a structure that generates electrical signals when exposed to light.

Embodiments are directed to the system of paragraph [0012], wherein the one or more low accuracy blood flow sensors generate images over a same period of time that the one or more high accuracy blood flow sensor measures a local blood flow at a reference site.

Embodiments are directed to the system of paragraph [0012], wherein the physical devices that include at least a structure that generates electrical signals when exposed to light are cameras and wherein the cameras are equipped with optical filtered.

Embodiments are directed to the system of paragraph [0006], wherein one or more of the high accuracy blood flow sensors and the one or more low accuracy blood flow sensors are cameras, and wherein at least one camera is equipped with an optical filter.

Embodiments are directed to the system of paragraph [0015], wherein the at least one camera is an IR camera.

Embodiments are directed to the system of paragraph [0015], wherein a light source is configured to illuminate a subject in a wavelength range corresponding to the transmitted wavelength range of the optical filter equipped on the at least one camera.

Embodiments are directed to the system of paragraph [0006], wherein the controller is a hardware device which generates a pulsatile blood perfusion map based on a local blood flow measurement, images of one or more reference sites on the body of the patient, and images of one or more imaging sites on the body of the patient.

In a further aspect, embodiments of the present disclosure relate to a method for measuring pulsatile blood perfusion maps, including: obtaining a reference blood volume waveform using one or more high accuracy blood flow sensors positioned at a reference site of a patient; simultaneously obtaining a second blood volume waveform of any region of interest of a patient's body using one or more low accuracy blood flow sensors; estimating an amplitude of the second blood volume waveform using the reference blood volume waveform; and generating a spatial map of the amplitude of the blood volume waveform, wherein the spatial map of the amplitude is proportional to a pulsatile perfusion map.

Embodiments are directed to the method of paragraph [0019], wherein the one or more high accuracy blood flow sensor is one or more selected from a group consisting of pulse oximeter, electrocardioagraph, arterial catheters, and camera-based photo-plethysmography device.

Embodiments are directed to the method of paragraph [0019], wherein one or more of the high accuracy blood flow sensors and the one or more low accuracy blood flow sensors are cameras, and wherein at least one camera is equipped with an optical filter.

Embodiments are directed to the method of paragraph [0019], wherein estimating the amplitude is performed by estimating the amplitude of the second blood volume waveform obtained from each pixel block of the camera sensor given the reference blood volume waveform recorded using the high accuracy blood flow sensor.

Embodiments are directed to the method of paragraph [0019], wherein when the one or more high accuracy blood flow sensors are pulse oximeters and the one or more low accuracy blood flow sensors comprise a video camera, and an amplitude of the second blood volume waveform obtained from each pixel block of the video camera sensor is estimated given the reference blood volume waveform recorded using a pulse oximeter.

Embodiments are directed to the method of paragraph [0019], further comprising removing errors from the blood perfusion map by decorrelating a pulse signal from an optical path associated with the one or more low accuracy blood flow sensors.Embodiments are directed to the method of paragraph [0019], further comprising removing errors from the blood perfusion map by using an optical flow algorithm which is invariant to brightness variations due to blood volume change in the region of interest.

Other aspects and advantages of the claimed subject matter will be apparent from the following description and the appended claims.

BRIEF DESCRIPTION OF DRAWINGS

Certain embodiments of the invention will be described with reference to the accompanying drawings. However, the accompanying drawings illustrate only certain aspects or implementations of the disclosure by way of example and are not meant to limit the scope of the claims.

FIG. 1 shows the variation of average SNR (in dB) per pixel block of the estimated blood perfusion map according to embodiments of the present disclosure.

FIG. 2 shows the corresponding per pixel block SNR of the estimated blood perfusion map using a camera-only method.

FIGS. 3.1, 4.1, 5.1, and 6.1 show the temporal variations of the average perfusion in various patients according to embodiments of the present disclosure.

FIGS. 3.2, 4.2, 5.2, and 6.2 provide representative blood perfusion images at the indicated time points, 50, 150, and 180 seconds for each of the respective patients from FIGS 3.1, 4.1, 5.1, and 6.1.

FIGS. 7-9, 10.1-10.4, and 11 provide various system configurations in accordance with embodiments of the present disclosure.

FIG. 12 is a flow diagram depicting methods in accordance with embodiments of the present disclosure.

DETAILED DESCRIPTION

Specific embodiments will now be described with reference to the accompanying figures. In the following description, numerous details are set forth as examples of the disclosure. It will be understood by those skilled in the art that one or more embodiments of the present disclosure may be practiced without these specific details and that numerous variations or modifications may be possible without departing from the scope of the disclosure. Certain details known to those of ordinary skill in the art are omitted to avoid obscuring the description.

Generally, embodiments disclosed herein relate to methods, systems, and devices for generating pulsatile blood perfusion maps. More specifically, embodiments disclosed herein relate to a multi-sensor imaging system for measuring pulsatile blood perfusion maps formed of a high accuracy blood flow sensor, a low accuracy blood flow sensor and a controller connected to the high accuracy blood flow sensor and the low accuracy blood flow sensor by at least one operable connection. In one or more embodiments, systems and methods in accordance with the present disclosure may include a multi-sensor modality for measuring blood perfusion by combining a high accuracy sensor (such as, for example, a pulse oximeter) with a low accuracy sensor (such as a video camera) to generate a blood perfusion map. In some embodiments, a signal model for blood perfusion imaging may account for differences in camera operating parameters, and may include a maximum likelihood (ML) estimator for estimating three-dimensional blood perfusion maps by combing measurements from a camera and a pulse oximeter.

As noted above, blood perfusion is the flow of blood to the end organs and tissues through the blood vessels in the body. As the blood perfusion is a pulsatile flow that changes with periodic variations, measuring the pulsatile blood perfusion in a certain region of the body generates a pulsatile blood perfusion map that provides information useful for diagnosing a variety of pathologies. Perfusion measurement is important to diagnose reference arterial disease (PAD) such as diabetic foot ulcers, to monitor perfusion in patients during surgery and to monitor critical care patients in Intensive Care Units (ICU). However, existing point modalities to measure blood perfusion such as laser Doppler flowmeter and perfusion index (measured using only a pulse oximeter) are not useful as they do not capture any spatial variations in blood perfusion, which may have diagnostic value.

Existing imaging modalities to measure blood perfusion such as laser speckle contrast imaging, laser Doppler imaging devices, and fluorescence angiography are commercially available for measuring blood perfusion maps. However, these devices (i) are bulky and require specialized measurement protocols, (ii) are not generally used in operating rooms or at the bedside in the intensive care units (ICUs) as they may cause interference to the ongoing care and discomfort to the patients, and (iii) are too expensive to be used routinely as a point-of-care device for patient diagnostics. Methods and systems in accordance with the present disclosure may reliably obtain spatial and temporal blood perfusion maps using a multi-sensor camera-based modality, which is low cost and easy to use.

Methods in accordance with the present disclosure may combine measurements from a system of measurement devices to provide a method of generating a high accuracy pulsatile blood perfusion map over a large area of the patient's body. Specifically, the multi-sensor imaging systems in accordance with the present disclosure may combine one or more disparate devices to improve the accuracy of the pulsatile perfusion map, and thereby makes it useful for clinical applications and suitable for further development as a clinical device.

Methods in accordance with the present disclosure may generate pulsatile blood perfusion maps by combining multiple sensor measurements of a patient. In one or more embodiments, an imaging system for determining pulsatile blood perfusion maps may include a number of sensors and one or more controllers, wherein the sensors and the controller may be linked by one or more operable connections. In some embodiments, sensor measurements may be obtained from different types of sensors. Combining multiple sensor measurements may improve the accuracy of the determined pulsatile blood perfusions maps when compared with blood perfusions maps derived from a single sensor or multiple sensors of the same type.

In various embodiments, a high accuracy blood volume sensor measures blood volume change at a reference location of a patient's body, generating a reference blood volume waveform that may be combined with any lower accuracy blood volume sensors to improve the overall accuracy of blood perfusion imaging. For example, a high accuracy blood volume sensor may be used to generate a first reference blood volume change waveform, which is then used to improve the accuracy of a second blood volume waveform obtained from a low accuracy blood volume sensor measuring blood volume at the same or separate region of a patient's body. In addition, the measurement obtained from the low accuracy blood volume sensor may contain information from an imaging area that is the same as the high accuracy blood flow sensor, or may contain information from a different area. In one or more embodiments, a high accuracy blood volume sensor may be placed at a reference site on a patient's body. As defined herein, a reference site is any external (or internal) part area of a patient's body, such as a finger or an ear lobe or toe or forehead (for a pulse oximeter), chest (for an electrocardiogram) or wrist, elbow, groin, or foot in the case of an arterial catheter. As defined herein, an imaging site of a patient's body is any external or internal body site over which blood perfusion maps need to be measured.

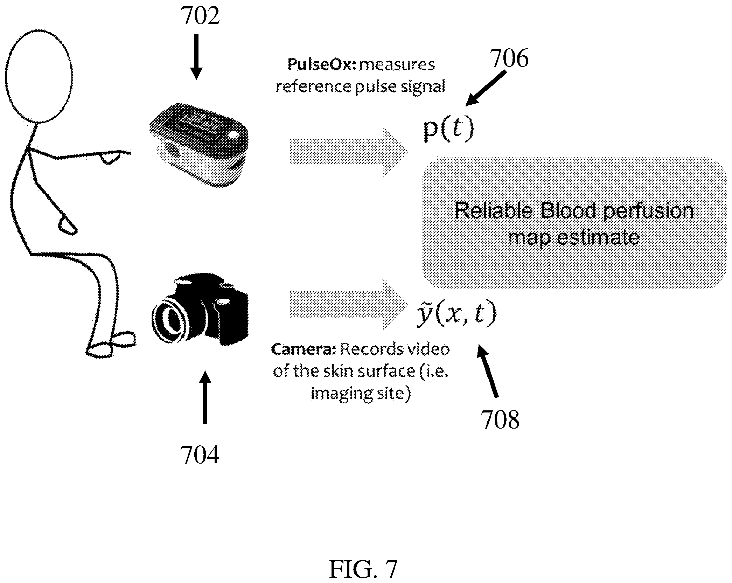

As noted above, the multi-sensor imaging systems in accordance with the present disclosure may combine one or more disparate devices and use a reliable blood volume waveform from a high accuracy blood volume sensor (such as a pulse oximeter) as a reference, and then correlating the reference waveform with the noisy blood volume waveform obtained from the low accuracy blood volume sensor, such as a camera. In some embodiments, a reference waveform is correlated with each pixel in the camera to produce accurate and high-resolution perfusion maps of any imaged skin surface. This configuration is discussed in greater detail below with respect to FIG. 7 in the Applications section.

In one or more embodiments, a high accuracy blood volume sensor may include a pulse oximeter that determines local blood volume change at a reference location, which may then be used to derive blood perfusion. The pulse oximeter may be a physical device that measures blood perfusion over a small area with high accuracy. The pulse oximeter may measure the local blood volume at the reference site over time and thereby enable a rate of change of blood volume which equals blood flow at the reference site to be determined.

It is also envisioned that other high accuracy sensors for measuring cardiac-related pulse signal at a reference site may be used instead of a blood volume sensor, such as an electrocardiograph or an arterial catheter to measure the pulse pressure waveform without departing from the present disclosure.

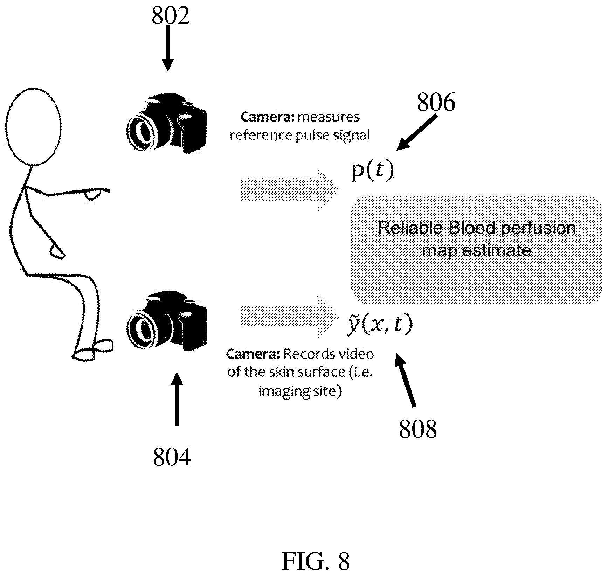

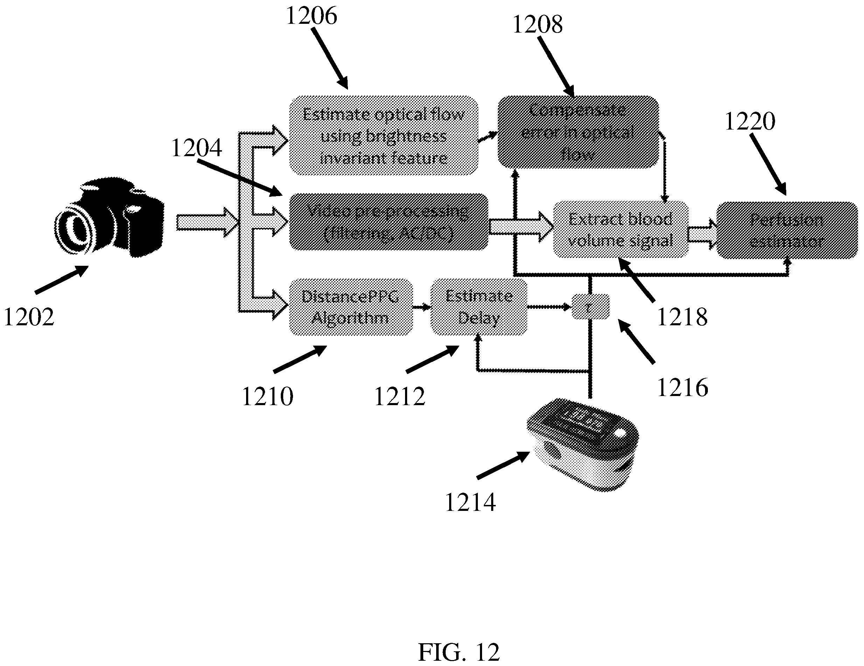

It is also envisioned that the high accuracy blood volume sensor can be another camera, or the same camera as the low accuracy blood flow sensor. The high accuracy blood sensor may be configured in some embodiments to record video of a reference skin surface that is used to generate a reliable blood volume waveform using a number of techniques including those described in U.S. Pat. Pub. 2016/0143538 A1, which is incorporated by reference in its entirety. This configuration is also discussed in greater detail below with respect to FIGS. 8 and 12 in the Applications section. In one or more embodiments, a single camera may function as both the high accuracy blood flow sensor and low accuracy blood flow sensor by selecting distinct regions on the camera viewing area. For example, a fraction of pixels on a CCD chip having acceptable SNR may be assigned to record the high accuracy blood measurement, while the remainder is devoted to one or more low blood flow sensing regions used to develop a target perfusion map.

In one or more embodiments, the low accuracy blood flow sensor may be a physical device, such as a video camera, that generates images of both the reference site and a plurality of imaging sites on the body of the patient. In such embodiments, the imaging sites may be internal or external sites of the body of the patient. The physical device may, for example, include a charge couple device or other structure that generates electrical signals, such as images, when exposed to light.

The images generated by the low accuracy blood flow sensor may be generated over the same period of time that the high accuracy blood flow sensor measures the local blood flow at the reference site. By generating images of a reference site over the same period of time that the high accuracy sensor is measuring the local blood flow, the images of the reference site may be correlated with the measured local blood flow at the reference site. Additionally, the pixels of the images of the generated images may provide a rough estimation of the blood flow of the patient at each location of the patient associated with each pixel.

Embodiments of the low accuracy sensor are not limited to a single camera. The low accuracy sensor may include any number of cameras that independently image portions of the patient's body. Additionally, the portions of the patient imaged are not restricted to external sites, e.g., the skin, of the patient. Cameras may be used to image internal portions of the patient such as, for example, organs or wound sites, or internal body organs like intestine, kidneys, lungs, heart, brain etc. using either laparoscopic cameras or during open surgery with externally mounted cameras without departing from the present disclosure. This configuration is discussed in greater detail below with respect to FIG. 7 in the Applications section.

It is also envisioned that the high accuracy blood flow sensor and/or low accuracy blood flow sensor may include a camera equipped with an optical filter. Optical filters are commercially available that select for any combination of working emission wavelength ranges such as a green, red, blue, infrared (IR), near-IR, and the like. Due to the different penetration depth of different wavelengths of light, utilizing different filters may enable blood perfusion at different depths to be estimated. In other words, different filters may enable tomographic rather than strictly topographic blood flow data to be generated. Moreover, the high accuracy blood flow sensor and the low accuracy blood flow sensors may each be a camera equipped with the same or different optical filter. In some embodiments, an appropriate excitation illumination source may be used to complement the filter placed before a camera functioning as a high accuracy blood flow sensor and/or low accuracy blood flow sensors. For example, when an IR filter is equipped on a camera, an IR illumination source may be used to enhance the signal received by the camera.

In one or more embodiments, the high accuracy sensor is a pulse oximeter, while the low accuracy sensor is a camera. Both the pulse oximeter and the camera measure the blood volume change over time at a reference site through optical means. A pulse oximeter is a simple spot measurement device which can measure blood volume waveform reliably from one body location, but cannot simultaneously take spatial measurements from a large region of the skin surface. On the other hand, a camera provides noisy measurements of blood volume waveform, but a camera may simultaneously take spatial measurements from a large region of the imaged skin surface owing to its unique spatial dimension: each pixel on the image sensor can be considered as a pulse oximeter which is virtually (from a distance) attached to the corresponding location on the imaged skin surface and provides an independent but noisy measurement of the blood volume waveform from that location.

In one or more embodiments, the multi-sensor imaging system may include at least a controller. According to the present embodiments, the controller may be a hardware device configured to generate the pulsatile blood flow maps based on the local blood flow measurement and the images of the reference site and imaging sites on the body of the patient. The controller may be connected to the high accuracy sensor and the low accuracy sensor by one or more operable connections.

In one or more embodiments, the hardware device may be, for example, an application specific integrated circuit, a digital signal processor, a programmable gate array, an integrated circuit, or a printed circuit board that includes circuitry. It is also envisioned that the hardware device may include a non-transitory computer readable medium that stores instructions that, when executed by a processor of the controller, cause the controller to perform the functions of the controller described herein.

According to the present embodiments, the controller may generate the pulsatile blood flow maps by obtaining the local blood flow measurement from the high accuracy sensor and the images generated by the low accuracy sensor. For example, the controller may obtain the measurements from the high accuracy sensor by way of operable connections. In another example, the controller may obtain the measurements and images from a storage operably connected to the controller on which the measurements and images are stored.

The controller as described herein may analyze the images obtained by the low accuracy blood flow sensor and may determine at least one pixel associated with the peripheral site where the high accuracy blood flow sensor performed blood flow measurement on the patient's body. The controller may analyze the images by, for example, using a predetermined spatial association between the generated images and the peripheral site. In another example, the controller may analyze the images using image recognition that identifies a known location on the patient's body and, based on the known location, identifies at least pixel of the images based on a predetermined spatial association between the known location and the reference site.

For example, when the high accuracy sensor is a pulse oximeter and the low accuracy sensor is a CMOS/CCD camera, the method for measuring pulsatile blood perfusion maps may includes the following algorithm: first, a reference blood volume waveform is obtained using a pulse oximeter from a reliable location on the body. Next, any region of interest (ROI), either on the skin surface or any internal tissue, is simultaneously video recorded using a CMOS/CCD camera. In such embodiment, an optical filter may be placed in front of the camera, such as a green optical filter. However, it is envisioned that other optical filters may be used, depending on the depth of the area that is targeted. Afterwards, the amplitude of the blood volume waveform obtained from each pixel block of the camera sensor is estimated given the reference blood volume waveform recorded using a pulse oximeter. As described later in greater detail, a maximum-likelihood estimator for amplitude as described by equation 4 may be used. In such embodiments, the spatial map of the amplitude of the blood volume waveform generated is proportional to the pulsatile perfusion map. By using the blood volume waveform obtained from a pulse oximeter as a reference, the accuracy of amplitude estimator is improved due to locked-in amplification. This configuration is discussed in greater detail below with respect to FIG. 7 in the Applications section.

It is also envisioned that a camera array may be used instead of a single camera, where each camera has a different optical filter (e.g. green, red, near infrared, etc.) placed in front of each camera to obtain pulsatile perfusion maps at different tissue depth. Longer wavelength of light penetrates deeper into the tissue. This has the potential to provide pulsatile perfusion tomography, rather than just the topography map obtained using single wavelength of light.

In one or more embodiments, the reference blood volume waveform may be estimated using a camera-based method (for example, using a camera-based Photoplethysmogram Estimation as discussed in U.S. Pat. Pub. No. 2016/0143538 A1, which is incorporated by reference in its entirety).

One limitation of current invention may be due to the penetration depth of visible and near-infrared light into tissue which is limited to few millimeters. In such a case, the modality as described herein may be limited to measuring peripheral perfusion just below the skin surface or internal tissue. This is considered acceptable for applications highlighted above, e.g., monitoring of patients in intensive care units, or for measuring peripheral perfusion to diagnose peripheral arterial disease or to monitor internal or external blood perfusion during a surgery or for measuring peripheral perfusion in intensive care unit. All other imaging modalities for measuring perfusion (e.g. laser speckle contrast imaging, laser Doppler perfusion imaging, or fluorescein angiography) may also suffer from similar limitation.

**Blood perfusion signal model**

As defined herein, blood flow (or perfusion) is the rate of change of blood volume. The volume of blood in the vessels generally changes in sync with the beating of the heart. As the heart rate remains more or less constant at homeostasis, the rate of change of blood volume is proportional to the amplitude of the blood volume waveform. Thus, to measure spatial blood perfusion map, the amplitude of the blood volume waveform is estimated at different tissue locations over time. In one or more embodiments, methods in accordance with the present disclosure may incorporate a blood perfusion signal model that generates blood volume waveforms from optical information, including sources such as cameras and other optical devices.

When light falls on the skin surface, it is partly absorbed by the skin and the underlying tissue, and is partly reflected back and recorded by the camera sensor imaging the skin surface. The incident light intensity $I(\vec{x})$ may or may not change over time. Here, $(\vec{x})$ denotes the location on the skin surface corresponding to pixel as defined in Eq. 1.

$$\vec{x} = \{x, y\} \qquad (1)$$

on the camera. Then, the camera recorded video signal over time can be modeled as Eq. 2, where the skin reflectance is separated into two components: a first component $b(\vec{x})$ that is due to light absorption by skin surface and tissue underneath and is time invariant, and the second component $c(\vec{x}, t)$ is due to light absorption by the chromophores in the blood, and is time varying due to pulsatile changes in the blood volume in the microvasculature underneath the skin surface. Finally, $w(\vec{x}, t)$ is the noise added during the camera acquisition process.

$$V(\vec{x}, t) = I(\vec{x})\big(b(\vec{x}) + c(\vec{x}, t)\big) + w(\vec{x}, t) \qquad (2)$$

The subsurface light absorption component due to pulsatile changes in blood volume can be decoupled as shown in Eq. 3, where $a(\vec{x}, t)$ is the amplitude of the blood volume waveform p(t) and is different at different location.

$$c(\vec{x}, t) = a(\vec{x}, t)p\big(t - \tau(\vec{x})\big) \qquad (3)$$

The amplitude $a(\vec{x}, t)$ can also change over time due to temporal variations in blood perfusion, but at a rate that will be much slower in comparison to the instantaneous variations in p(t) which is in sync with the beating of the heart. The blood volume waveform signal is assumed to be delayed by different time $\tau(\vec{x})$ at different locations $\vec{x}$ on the skin surface. The noise term $w(\vec{x}, t)$ is dominated by (i) camera's quantization noise, readout noise and photon shot noise, and motion artifact. Taking camera parameters also into account, the camera-recorded video signal can be modeled as Eq. 4, where Q is the multiplication factor due to camera's exposure and aperture settings, $w_c(t)$ is the noise added due to camera's acquisition process, and $w_m(t)$ is the motion artifact. Also, to simplify the model, the spatial variation in light intensity is assumed to be minimal, and is replaced with mean illumination value $I_0$ over the imaged skin region.

$$V(\vec{x}, t) = QI_0\big(b(\vec{x}) + a(\vec{x}, t)p\big(t - \tau(\vec{x})\big)\big) + w_c(t) + w_m(t) \qquad (4)$$

Based on the proposed signal model, an estimator for the blood perfusion map $a(\vec{x}, t)$ was developed given a noisy camera recording $V(\vec{x}, t)$, and the underlying blood volume waveform signal p(t) which is reliably measured using a pulse oximeter.

**Blood perfusion estimation**

Blood perfusion estimation using a multi-sensor imaging system in accordance with the present disclosure may involve a series of two preprocessing stages to be performed on the raw video recording from an optical device. After the preprocessing stage, the processed perfusion signal from the camera and pulse oximeter recordings are fused together to estimate the blood perfusion map.

First the raw video measurement $V(\vec{x}, t)$ from the camera is spatially blurred using a Gaussian Blurring filter (Size:5) and each image of the video is resized, horizontally and vertically, by a factor of ½. This reduces the impact of camera measurement noise to both optical flow (motion) and perfusion estimate. Then, the error-corrected optical flow path $\vec{x}(t)$ is obtained as detailed in the motion compensation section below. The raw perfusion signal $r^{raw}(\vec{x}, t)$ is obtained by spatially averaging the camera measurement over MxM pixel block along the obtained optical flow path $\vec{x}(t)$. The choice of M will be a trade-off between the spatial resolution of resulting perfusion map and the desired SNR for the perfusion estimate per MxM pixel block.

Second, the raw measurement at each skin site $\vec{x}$ is normalized (for illumination invariance) as "AC over DC", where AC stands for the cardiac-related pulsatile time- varying change in skin reflectance which is obtained using a bandpass filter (0.5 Hz-5Hz) and the DC component is obtained using a lowpass filter (cutoff frequency 0.3 Hz), *i.e.*, $r^{AC/DC}(\vec{x}, t) = r^{BF}(\vec{x}, t)/r^{LF}(\vec{x}, t)$.

Since the noise in the camera measurement is white and Gaussian, the maximum likelihood estimator for the perfusion $a(\vec{x}, t)$ is defined by Eq. 5 where $<\cdot, \cdot>$ is the inner product between vectors. The delay $D(\vec{x})$ is used to align the reference blood volume waveform signal p(t) with the blood volume waveform signal at location $\vec{x}$. The inner product in the above equation is defined over the time window T over which the perfusion need to be determined.

$$a(\vec{x},t)_{ML} = \langle r^{AC/DC}(\vec{x},t), p(t - D(\vec{x})) \rangle \qquad (5)$$

The signal-to-noise ratio of the above ML estimate is same as the SNR of the signal of interest and can be estimated as shown in Eq. 7:

$$SNR(\vec{x},t) = \frac{\hat{a}^2_{ML}(\vec{x},t)}{\widehat{Var}\left(V_N(\vec{x},t) - \hat{a}_{ML}(\vec{x})p(t - D(\vec{x}))\right)_T} \qquad (6)$$

**Motion Compensation**

As the imaged skin surface can move during camera recording, there is a need to establish correspondence between each pixel in the acquired image (camera's frame of reference) to points on the imaged skin surface (subject's frame of reference). Standard optical flow algorithms can be used to find this correspondence, and obtain the path $\vec{x}(t)$ that a point $\vec{x}$ on the skin surface traverses. For obtaining perfusion estimate at location $\vec{x}$ on the skin surface, the perfusion measurement should then be done along the path $\vec{x}(t)$ in the camera's reference frame. Most optical flow algorithm assumes brightness constancy to find pixel correspondence from one frame to the next, i.e. they assumed that $y(\vec{x}(t + 1), t + 1) = y(\vec{x}(t), t)$. But, in blood perfusion imaging the appearance (or brightness) of the skin surface can changes due to blood volume change underneath. Consequently, the brightness constancy assumption inherent in optical flow algorithms is invalid in the context of blood perfusion imaging. Therefore, conventional optical flow algorithm if used as-is results in erroneous optical flow path $\hat{x}(t)$ and results in corrupted blood perfusion estimate.

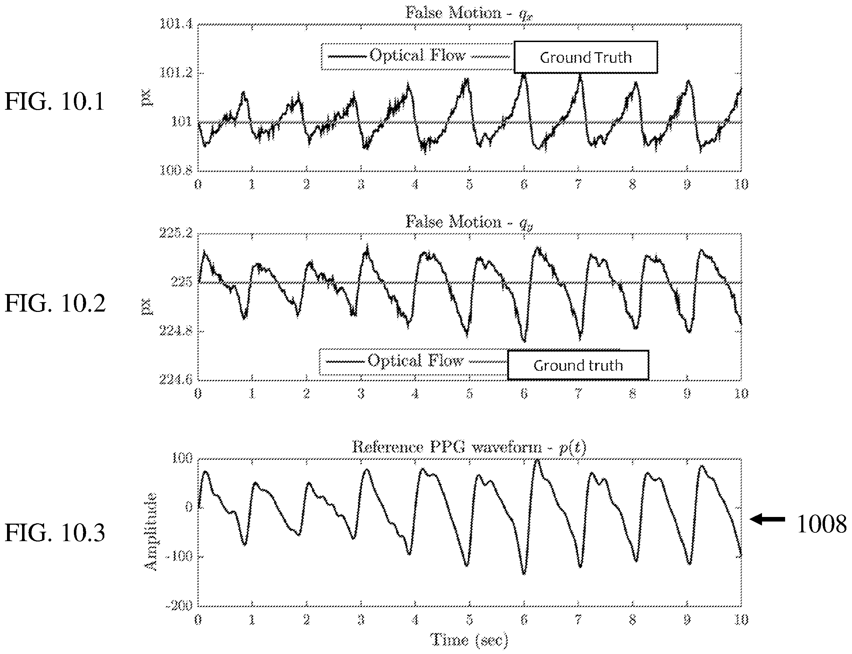

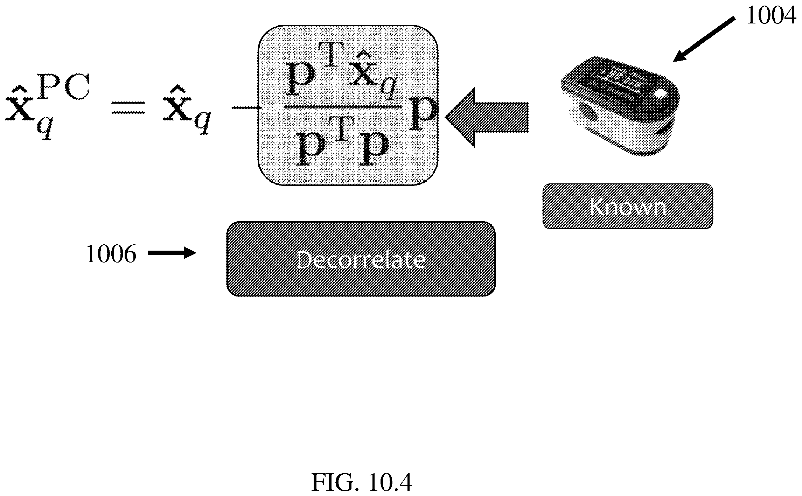

In one or more embodiments of the current invention, the error in the optical flow path (obtained using any conventional optical flow algorithm) can be removed as the error is proportional to the reference pulse waveform. Therefore, the error in the optical flow path can be removed by way of decorrelating the pulse signal from the obtained erroneous optical flow path. Once the error in optical flow is removed, then a reliable blood perfusion maps can be obtained using the corrected optical flow path. This configuration is discussed in greater detail below with respect to FIG. 10 in the Applications section.

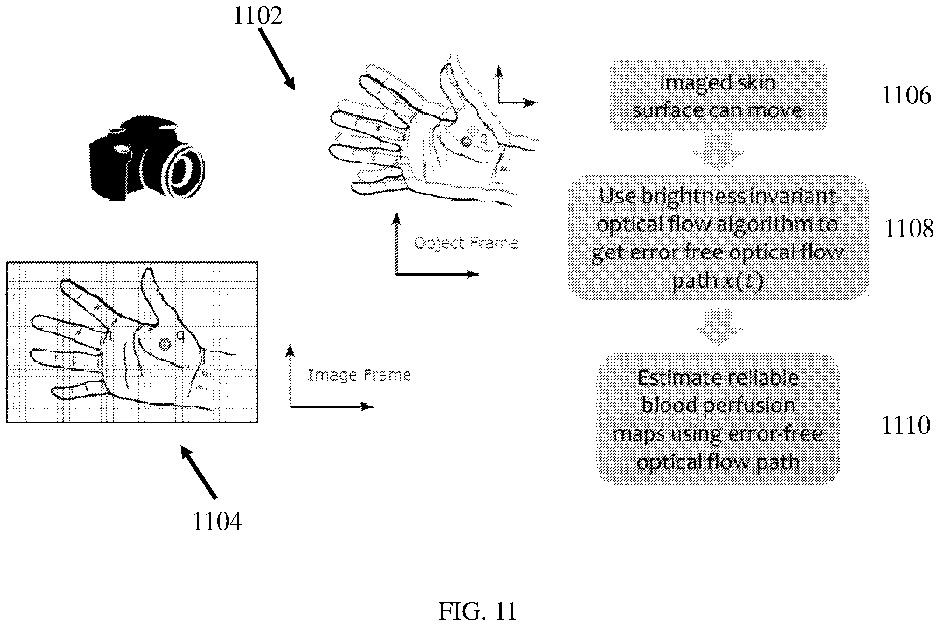

In one or more embodiments of the current invention, any special optical flow algorithm can be used which is invariant to brightness variations due to blood volume change in the imaged skin surface to obtain correct optical flow paths. Brightness invariant optical flow algorithms like those which are based on matching spatial gradient features, or those which match texture of the image, or those which match brightness invariant mean-subtracted normalized cross correlation, census transform, or mean-subtracted sum of absolute differences, or any other brightness invariant feature can be used to obtain correct optical flow path. Once the error-free optical flow path is obtained using brightness invariant optical flow method, then a reliable blood perfusion maps can be obtained using the error-free optical flow path. This configuration is discussed in greater detail below with respect to FIG. 11 in the Applications section. In one or more embodiments, a controller may, using the determined optical flow path of each pixel of the images, determine the local blood flow in each of the imaged sites of the patient.

The disclosed multi-sensor modality may improve per pixel signal to noise ratio of the perfusion map by up to 3 dB in some embodiments. For example, multi-sensor methods have been used to produce perfusion maps with 2-3 times better spatial resolution over comparative camera-only methods. Blood perfusion measured in the palm using the disclosed multi-sensor imaging system during a post-occlusive reactive hyperemia (POHR) test replicates data using existing laser Doppler perfusion monitor but with much lower cost and a portable setup making it suitable for further development as a clinical device. In one or more embodiments, multi-sensor imaging systems in accordance with the present disclosure may involve minimal spatial averaging over 4x4 pixel blocks, and produce blood perfusion maps with 0.5-3 dB higher signal to noise ratio (SNR) per pixel block compared to camera-only techniques to generate blood perfusion maps. As shown in the following examples, multi-sensor functionality was validated by conducting a standardized post-occlusive reactive hyperemia (POHR) test on 4 healthy individuals and found the derived blood perfusion measurements to be in agreement with published POHR-test response curve measured using a laser Doppler perfusion monitoring device.

**Examples**

The following examples are presented to further illustrate the properties of multi-sensor imaging systems in accordance with the present disclosure, and should not be construed to limit the scope of the disclosure, unless otherwise expressly indicated in the appended claims.

Two sets of experiments were presented. In the first set, a controlled experiment to characterize the average SNR per pixel block for blood perfusion imaging using the multi-sensor imaging system as described herein and using camera-only method as a function of ADC quantization level and spatial mean filter size M were used. In the second set of experiments, a standard post occlusive reactive hyperemia (POHR) test was performed on 4 healthy individuals to measure the change in their blood perfusion in the palm before, during and after an occlusion event.

The experimental setup involves of a monochromatic CMOS camera (Grasshopper GS3-U3-23S6M-C from Point Grey) on which a green optical filter having an optical passband between 520 nm to 560 nm was placed. Using green optical filter improves the SNR of camera based blood volume waveform as the absorption spectra of hemoglobin peaks at a wavelength of around 530 nm. The camera is operated at 30 fps, with automatic gain control and gamma correction turned off, and exposure time is set at 12 ms. For all the experiments, the camera records a video of the palm rested on a hand support. Occlusion of blood flowing to the palm is done using a standard pressure cuff put on the arm of the same hand. A blood volume waveform was simultaneously recorded from the middle finger of the other hand using Biopac system's MP150 data acquisition unit as a reference pulse oximeter.

*(a) Perfusion imaging SNR*

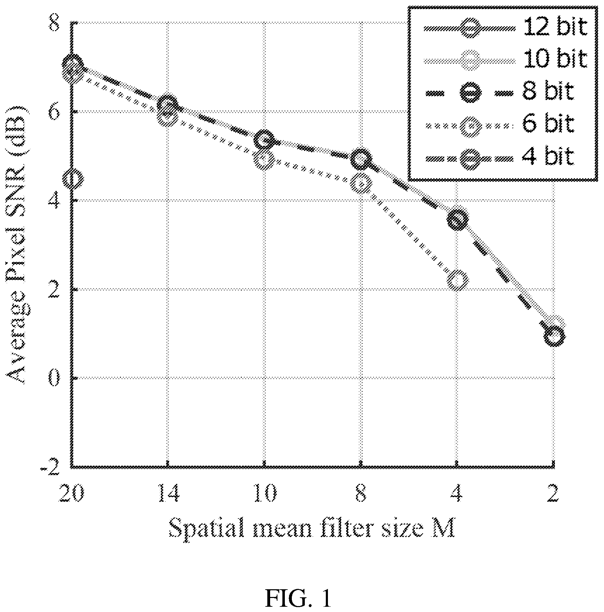

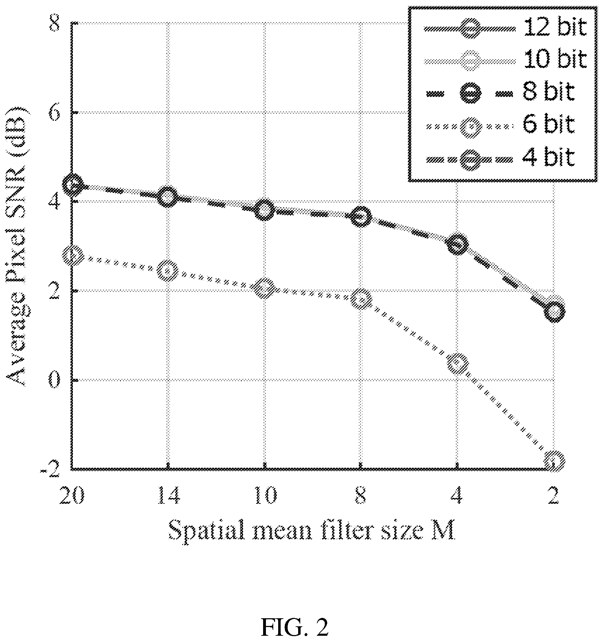

Referring now to FIG. 1, the variation of average SNR (in dB) per pixel block (computed using Equation (6)) of the estimated blood perfusion map using the multi-sensor imaging system as described herein as spatial averaging filter size $M^2$ is varied from $20 \times 20$ down to $2 \times 2$ is shown. FIG. 2 shows the corresponding per pixel block SNR of the estimated blood perfusion map using the camera-only method. The time window T for perfusion estimate is set to 10 sec for both methods. During this controlled experiment, the motion artifact due to hand movement is kept small so that the source of noise is due to the camera acquisition process. On an average, an SNR improvement of 0.5- 3 dB per pixel block was observed in the blood perfusion map derived from the multi-sensor as described herein compared to camera-only method.

*(b) POHR test*

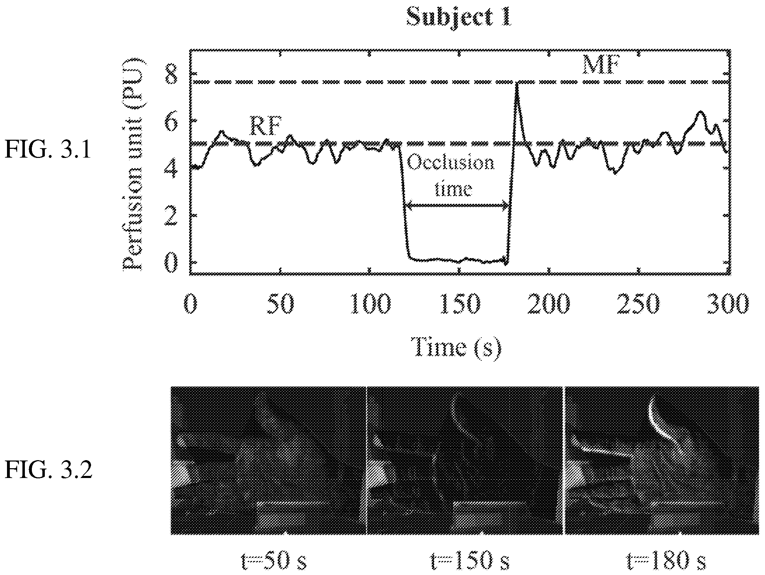

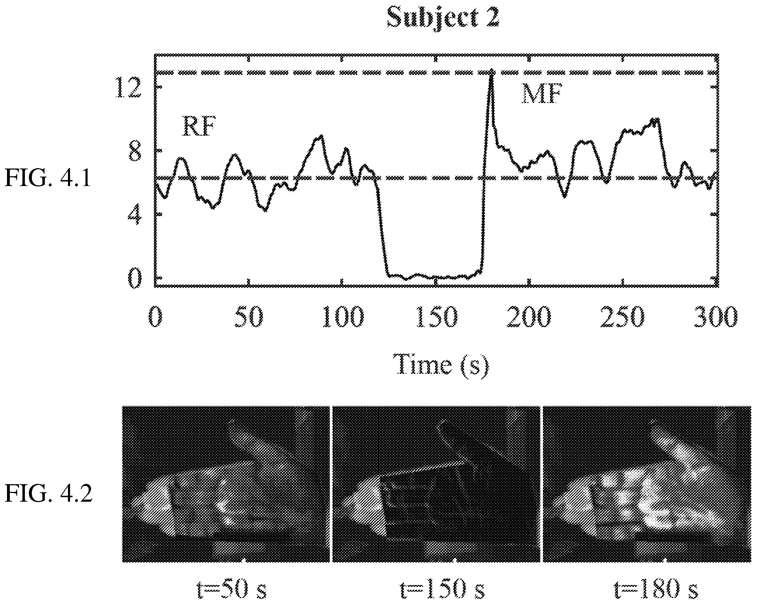

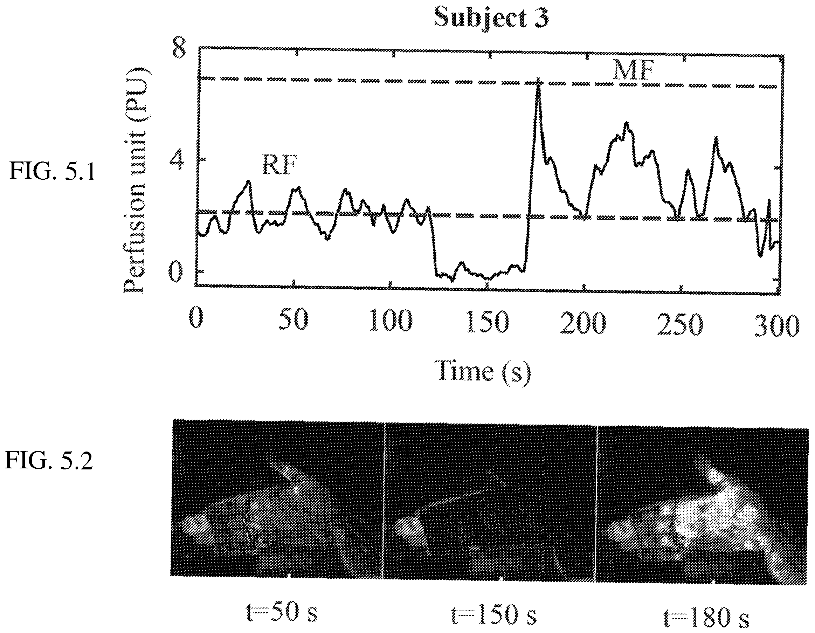

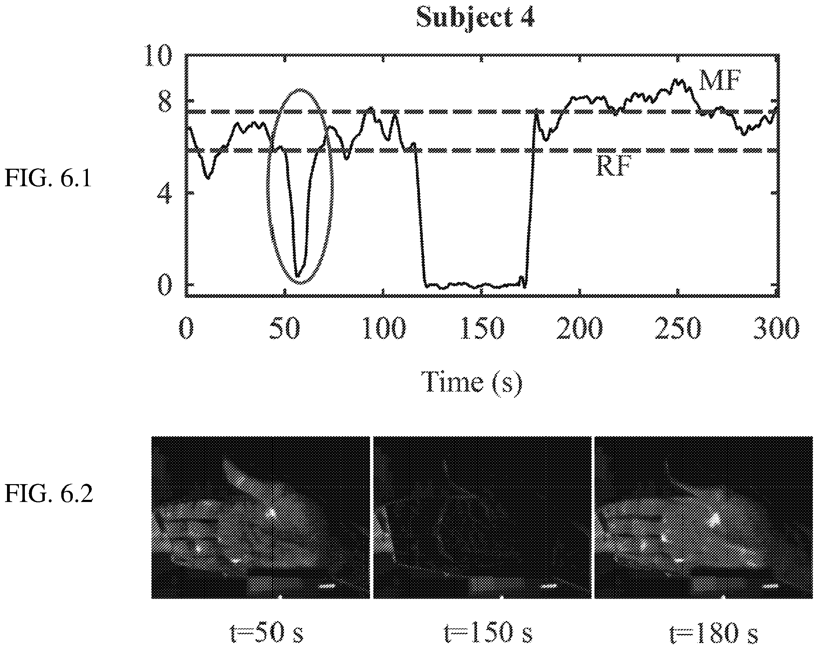

For this experiment, 2 min video of the palm before the occlusion was recorded to obtain a baseline estimate of perfusion, a total of 1 min video under occlusion, and a 2 min video post occlusion for 4 individuals having different skin tones (1 Caucasian, 1 Asian, and 2 brown). Referring to FIGS 3.1, 4.1, 5.1, and 6.1, temporal variation of the average blood perfusion in the palm of 4 subjects is shown before, during and after an occlusion test, RF is the resting flux (perfusion) before occlusion, MF is the maximum flux just after the occlusion. The temporal variations of the average perfusion in the palm (averaged over all pixel block in the palm region excluding fingers) during the POHR test for 4 healthy individuals is shown before, during and after the occlusion. The time window T for perfusion estimate is set to 5 sec with a 4 sec overlap (sliding window) to track sudden changes in perfusion due to occlusion. FIGS. 3.2, 4.2, 5.2, and 6.2 provide representative optical flow images at the indicated time points, 50, 150, and 180 seconds for each of the respective patients from FIGS 3.1, 4.1, 5.1, and 6.1. Referring now to FIG. 6.1, the sudden dip in estimated blood perfusion inside the marking (an oval is used to highlight this feature for Subject 4) is due to recording artifact in the reference pulse oximeter derived blood volume waveform.

These POHR response curve agrees well with POHR curve estimated using laser Doppler perfusion monitoring. The average perfusion before the occlusion (marked as RF) is lower than the average perfusion just after the release of the occlusion (marked as MF), and the ratio MF/RF also has diagnostic value for assessing arterial health. The variations in the average perfusion around the baseline could be due to rhythmic oscillations in the vascular tone caused by changes in smooth muscle constriction and dilation, and are 4-10 cycles per minute (cpm). For subject 3, the sudden dip in estimated blood perfusion at around t = 50 sec is due to recording artifact in the reference pulse oximeter derived blood volume waveform.

Adjacent to the POHR response curve of each subject is an image inlet showing the two dimensional spatial map of the blood perfusion at specific times (marked as dashed line with arrow) during the occlusion and release cycle. These spatial maps are generated with $M^2 = 4 \times 4$ spatial averaging filter. Darker pixel blocks in the spatial map show lower blood perfusion, whereas brighter pixel blocks show higher blood perfusion. There are spatial variations in the recorded blood perfusion e.g. palm regions around the base of the fingers shows higher perfusion, whereas regions around the hand marking in the palm shows lower blood perfusion. Similar conclusions are difficult to draw from noisy blood perfusion maps generated using camera-only methods. Using the multi-sensor imaging system as described herein, one may also visualize the dynamics of blood flow using temporal video of blood perfusion maps in the hand before, during and after the occlusion.

**Applications**

The multi-sensor imaging systems as described herein may be used as a portable and low cost blood perfusion monitoring system. This could have applications in monitoring wound healing in diabetic patients, in plastic surgery to monitor skin flap perfusion after microvascular reconstructive procedure, and to assess the skin's endothelial function. In many scenarios, like in intensive care unit (ICU) and in operating rooms (OR), existing systems like laser Doppler imaging cannot be readily used, whereas the claimed multi-sensor system, by virtue of being passive and operable from a distance, is suited for such scenarios. This may open up the possibility of realtime blood perfusion and micro-circulatory monitoring at the bedside during surgery and in ICU care.

Referring now to FIG. 7, a system configuration in accordance with the present disclosure is presented. A high accuracy imaging device 702 is affixed to a patient at a reference site to establish a reference pulse signal. One or more low accuracy imaging devices 704 are attached at secondary measurement sites of interest. The reference blood perfusion is established at 706 and used in conjunction with the imaging information obtained from the secondary measurement sites at 708. The information from 706 and 708 are then combined using techniques in accordance with the present disclosure to establish a pulsatile blood perfusion map.

Referring now to FIG. 8, an additional system configuration in accordance with the present disclosure is presented. A low accuracy imaging device 802 is affixed to a patient at a reference site to establish a reference pulse signal. In this case, the reference site may be a site in which the pulsatile information is less likely to contain errors such as an internal site, or an area of the body with a high volume of near-surface blood flow such as a wrist or hand. One or more additional low accuracy imaging devices 804 are then attached to secondary measurement sites of interest. The reference blood perfusion is established at 806 and used in conjunction with the imaging information obtained from the secondary measurement sites at 806. The information from 806 and 808 are then combined using techniques in accordance with the present disclosure to establish a pulsatile blood perfusion map.

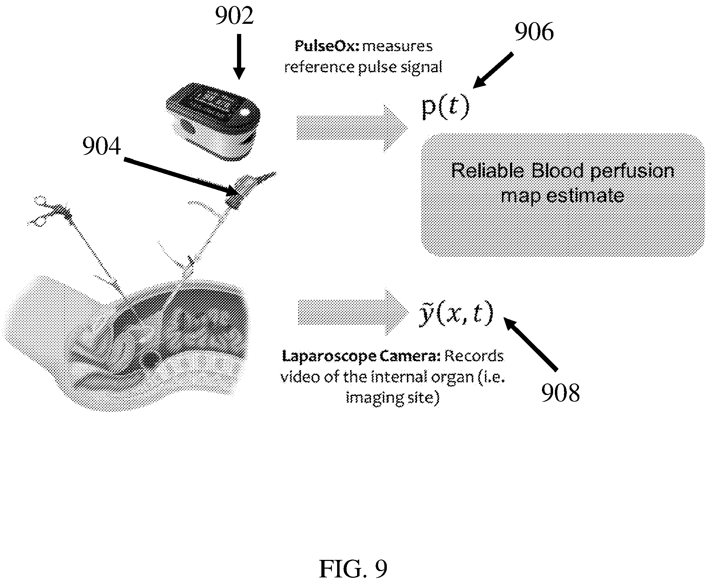

Referring now to FIG. 9, an additional system configuration in accordance with the present disclosure is presented. A high accuracy imaging device 902 is affixed to a patient at a reference site to establish a reference pulse signal. One or more additional low accuracy imaging devices 904 are then focused on secondary measurement sites of interest, in this case using a laparoscope or other suitable endoscope to view one or more internal sites of the body. The reference blood perfusion is established at 906 and used in conjunction with the imaging information obtained from the secondary measurement sites at 906. The information from 806 and 808 are then combined using techniques in accordance with the present disclosure to establish a pulsatile blood perfusion map.

Referring now to FIGS. 10.1-10.4, an embodiment in which an optical flow from a low accuracy image device is corrected using the input from a reference device such as a pulse oximeter. With particular respect to FIG. 10.1, the x-component of an optical flow (black trace) obtained from a low accuracy blood flow sensor is plotted as a function of time. Similarly, the y-component of the optical flow is represented in FIG. 10.2. The ground truth optical flow (gray trace) is also shown overlaid on the optical flow information in both FIGS. 10.1 and 10.2 respectively, which is the true motion of a point on a patient, defined as q. The error in optical flow due to the brightness constancy assumption is proportional to the pulse signal p(t) shown in FIG. 10.3. The error in optical flow is also referred to as false motion here.

With particular reference to FIG. 10.4, a reference measurement from known source 1004, such as a pulse oximeter, may then be used at 1006 to decorrelate the false motion from the optical flow signal obtained from the lower accuracy blood flow sensor and improve the accuracy of the resultant pulsatile perfusion map. Here, $\hat{X}_q$ is the erroneous optical flow vector of any point q on the imaged skin surface which is obtained from a low accuracy blood flow sensor

(e.g., a camera), $\hat{\mathbf{X}}_q^{\mathrm{PC}}$ is the corrected optical flow vector of the point q on the imaged skin surface, and **p** is pulse signal vector obtained from the reference high accuracy blood flow sensor, such as a pulse oximeter 1004. The correct optical flow path is then used to construct a blood perfusion map in accordance with methods of the present disclosure.

Referring now to FIG. 11, an embodiment in which motion artifacts are removed from an unsteady subject at 1102. As shown in the flow chart in FIG. 11, beginning at 1106, when a patient or subject moves, the imaged skin surface moves as well. To counter this a brightness invariant optical flow algorithm is used to get error free optical flow path x(t). The error free optical flow path is then used in 1110 to construct a blood perfusion map in accordance with methods of the present disclosure.

In order to provide a snapshot of a possible execution of a method in accordance with the present disclosure a flow diagram is presented in FIG. 12. Referring now to FIG. 12, a low accuracy imaging device 1202 such as a camera or camera array is used to measure the blood flow at a site on a patient. The video recording received from the low accuracy imaging device 1202 may then be preprocessed using various filtering techniques and/or perform illumination normalization using the AC/DC ratio at 1204. The blood volume signal is then extracted from the video recording using techniques in accordance with the present disclosure at 1218. A reference measurement obtained from a high or low accuracy imaging device 1214 is then used to determine the delay $\tau$ at 1212. The delay $\tau$ is the time delay between the high accuracy and low accuracy blood flow measurement and is used to time-align the reference blood flow measurement at 1216. The time aligned reference blood flow measurement from high accuracy blood flow sensor 1214 is then used to estimate the perfusion map at 1220 from the site of the low accuracy imaging device 1202. The perfusion measurements may then be collected to establish a pulsatile blood perfusion map.

If errors are detected in the optical flow received from low accuracy imaging device 1202, the optical flow may be corrected using a brightness invariant feature at 1206 and then any remaining error may be compensated at 1208 using temporal information from 1216 from high accuracy imaging device 1214 in accordance with the methods of the present invention. The method may then continue from 1218 as described above.

In another embodiment, the low accuracy imaging device 1202 such as a camera or a camera array can also be used to obtain the reference high accuracy blood flow signal, and therefore 1214 and 1202 are the same device in this embodiment, i.e. a camera or a camera array. To obtain high accuracy reference blood flow signal from a low accuracy device, we can select regions of high SNR from the imaged skin surface. One possible approach to select regions of high SNR is using the DistancePPG algorithm discussed in U.S. Pat. Pub. 2016/0143538 A1, which is incorporated by reference in its entirety. The high accuracy reference blood flow signal is then used to estimate the perfusion map at 1220 from the site of the low accuracy imaging device 1202. The perfusion measurements may then be collected to establish a pulsatile blood perfusion map.

One or more embodiments of the present disclosure may provide the following advantages: a system as described herein may provide a portable and low cost blood perfusion monitoring system. In addition, a system in accordance with the present embodiments may provide: 1) real-time pulsatile blood perfusion and microcirculation monitoring at the bedside in the ICU for critical care patients; 2) monitoring of wound healing, *e.g.*, in diabetic patients; 3) monitoring of skin flap blood perfusion after microvascular reconstructive procedure, *e.g.*, in plastic surgery patients; 4) monitoring of the level of anesthesia of a patient during surgery by measuring reference blood perfusion in different body locations, *e.g.*, face, hand, feet, *etc.*; 5) monitoring of blood perfusion of internal organ tissue during and/or after surgery, *e.g.*, gastrointestinal tract surgical patients.

While the invention has been described above with respect to a limited number of embodiments, those skilled in the art, having the benefit of this disclosure, will appreciate that other embodiments can be devised which do not depart from the scope of the invention as disclosed herein. Accordingly, the scope of the invention should be limited only by the attached claims.

**Claims**

1. A multi-sensor imaging system for generating a pulsatile blood perfusion map, comprising:

   a high accuracy blood flow sensor (702; 802) configured to generate a reference blood volume waveform at a reference site of a patient's body (706; 806), wherein the high accuracy blood flow sensor (702; 802) is a pulse oximeter;
   one or more low accuracy blood flow sensors (704; 804) configured to simultaneously generate a second blood volume waveform at any region of interest of the patient's body (708; 808), wherein the one or more low accuracy blood flow sensors (704; 804) are one or more camera sensors each equipped with an optical filter; and
   a controller connected to the high accuracy blood flow sensor (702; 802) and the one or more low accuracy blood flow sensors (704; 804) by at least one operable connection, wherein the controller is configured to generate the pulsatile blood perfusion map at a tissue depth by time-aligning the reference blood volume waveform with the second blood volume waveform and using the reference blood volume waveform obtained from the high accuracy blood flow sensor (702; 802) to improve the accuracy of the second blood volume waveform obtained from the one or more low accuracy blood flow sensors (704; 804).

2. The multi-sensor imaging system of claim 1, wherein the one or more low accuracy blood flow sensors are configured to measure blood flow over reference and/or imaging sites of the body of the patient.

3. The multi-sensor imaging system of claim 2, wherein the imaging sites are internal or external sites of the body of the patient.

4. The multi-sensor imaging system of claim 1, wherein the one or more low accuracy blood flow sensors are configured to generate images over a same period of time that the high accuracy blood flow sensor measures a local blood flow at a reference site.

5. The multi-sensor imaging sensor of claim 1, wherein the one or more camera sensors is an IR camera.

6. The multi-sensor imaging sensor of claim 1, wherein a light source is configured to illuminate a subject in a wavelength range corresponding to the transmitted wavelength range of the optical filter(s) equipped on the one or more camera sensors.

7. The multi-sensor imaging system of claim 1, wherein the controller is a hardware device configured to generate a pulsatile blood perfusion map based on a local blood flow measurement, images of one or more reference sites on the body of the patient, and images of one or more imaging sites on the body of the patient.

8. A method for generating pulsatile blood perfusion maps, comprising:

   obtaining a reference blood volume waveform using a high accuracy blood flow sensor (702; 802) positioned at a reference site of a patient's body (706; 806), wherein the high accuracy blood flow sensor (702; 802) is a pulse oximeter;
   simultaneously obtaining a second blood volume waveform of any region of interest of the patient's body (708; 808) using one or more low accuracy blood flow sensors (704; 804), wherein the one or more low accuracy blood flow sensors (704; 804) are one or more camera sensors each equipped with an optical filter;
   estimating an amplitude of the second blood volume waveform using the reference blood volume waveform; and generating a spatial map of the amplitude of the blood volume waveform, wherein the spatial map of the amplitude is proportional to a pulsatile blood perfusion map at a tissue depth,
   wherein the method further comprises time-aligning the reference blood volume waveform with the second blood volume waveform and using the reference blood volume waveform obtained from the high accuracy blood flow sensor (702; 802) to improve the accuracy of the second blood volume waveform obtained from the one or more low accuracy blood flow sensors (704; 804).

9. The method of claim 8, wherein estimating the amplitude is performed by estimating the amplitude of the second blood volume waveform obtained from each pixel block of the camera sensor given the reference blood volume waveform recorded using the high accuracy blood flow sensor.

10. The method of claim 8, further comprising removing errors from the blood perfusion map by decorrelating a pulse signal from an optical path associated with the one or more low accuracy blood flow sensors.

11. The method of claim 8, further comprising removing errors from the blood perfusion map by using an optical flow algorithm which is invariant to brightness variations due to blood volume change in the region of interest.

FIG. 1

EP 4 260 801 A2

FIG. 2

FIG. 3.1

FIG. 3.2

EP 4 260 801 A2

EP 4 260 801 A2

## Subject 2

FIG. 4.1

FIG. 4.2

t=50 s      t=150 s      t=180 s

## Subject 3

FIG. 5.1

FIG. 5.2

t=50 s          t=150 s          t=180 s

EP 4 260 801 A2

Subject 4

FIG. 6.1

Time (s)

FIG. 6.2

t=50 s          t=150 s          t=180 s

EP 4 260 801 A2

**702**

**PulseOx:** measures
reference pulse signal

**706**

$$p(t)$$

Reliable Blood perfusion
map estimate

$$\tilde{y}(x, t)$$

**Camera:** Records video
of the skin surface (i.e.
imaging site)

**708**

**704**

FIG. 7

FIG. 8

**FIG. 9**

FIG. 10.1

False Motion - $q_x$

FIG. 10.2

False Motion - $q_y$

FIG. 10.3

Reference PPG waveform - $p(t)$

1008

$$\hat{\mathbf{x}}_q^{\mathrm{PC}} = \hat{\mathbf{x}}_q - \frac{\mathbf{p}^{\mathrm{T}}\hat{\mathbf{x}}_q}{\mathbf{p}^{\mathrm{T}}\mathbf{p}}\mathbf{p}$$

1004

Known

1006 → Decorrelate

FIG. 10.4

**FIG. 11**

EP 4 260 801 A2

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160143538 A1